# EUROPEAN PATENT SPECIFICATION

(11) **EP 4 523 647 B1**
(45) Date of publication and mention of the grant of the patent: **22.07.2026**
(21) Application number: 23803793.1
(22) Date of filing: 09.05.2023
(51) Int. Cl.: A61B 34/20, A61B 90/00, A61B 6/00, A61B 17/00, A61B 6/03, A61B 6/12, A61B 6/40, A61B 6/46, A61B 6/58

(54) **METHOD AND SYSTEM FOR DETERMINING LOCATION OF SUBJECT OR SURGICAL TOOL**
VERFAHREN UND SYSTEM ZUR BESTIMMUNG DER POSITION EINES SUBJEKTS ODER EINES CHIRURGISCHEN WERKZEUGS
PROCÉDÉ ET SYSTÈME DE DÉTERMINATION DE L'EMPLACEMENT D'UN SUJET OU D'UN OUTIL CHIRURGICAL

(30) Priority: 10.05.2022 KR 20220057114
(43) Date of publication of application: 19.03.2025
(73) Proprietor: Koh Young Technology Inc., Seoul 08588 (KR)
(72) Inventor: ROH, Young Jun, Suwon-si Gyeonggi-do 16504 (KR); KIM, Young Kwang, Anyang-si Gyeonggi-do 13964 (KR); JO, Eun Ha, Gwangmyeong-si Gyeonggi-do 14313 (KR); OH, Tae Seok, Yongin-si Gyeonggi-do 16908 (KR); JEONG, Pil Soo, Yongin-si Gyeonggi-do 16812 (KR)
(74) Representative: Meissner Bolte Partnerschaft mbB
(86) International application number: PCT/KR2023/006238
(87) International publication number: WO 2023/219370

(56) References cited:
- WO-A2-2020/185048
- KR-A- 20140 120 145
- KR-A- 20170 124 962
- KR-A- 20170 124 962
- KR-A- 20180 115 122
- KR-A- 20190 123 865
- KR-A- 20220 018 986
- US-A1- 2006 235 287
- US-B1- 6 484 049

## Description

### TECHNICAL FIELD

The present disclosure relates to a method and a system for determining a location of a subject or surgical tool.

This study is a result of research conducted by the Ministry of Trade, Industry and Energy and the Korea Institute for Advancement of Technology under "Medium-sized Enterprise DNA Convergence Industry-Academic Cooperation Project". [Project name: AI-based low-dose multi-light source C-arm CT technology development for surgery, Project number: P0021346]

### BACKGROUND

In order to identify a location of an affected area of a subject (e.g., a patient) in a three-dimensional space, a three-dimensional medical image, such as a CT (Computed Tomography) image or an MRI (Magnetic Resonance Imaging) image, may be captured in advance. Afterwards, a doctor performs a surgery on the subject by referencing the pre-captured three-dimensional medical image. In order to accurately identify the affected area of the subject, an X-ray image may be further utilized.

US2006235287A1 may be considered to disclose a system for determining a location of a subject or a surgical tool, the system comprising: an X-ray device comprising a plurality of X-ray sources configured to irradiate the subject with X-rays and an X-ray detector configured to detect X-rays transmitted through the subject; a memory storing respective location coordinates of the plurality of X-ray sources and the X-ray detector in a first coordinate system of the system; and a processor configured to: obtain a three-dimensional medical image of the subject from an external device; obtain a plurality of first X-ray images of the subject using the X-ray device; match a second coordinate system of the three-dimensional medical image to the first coordinate system based on the plurality of first X-ray images, and the three-dimensional medical image; determine the location of the subject in the first coordinate system based on the matching result; obtain a plurality of second X-ray images of the surgical tool using the X-ray device.

### SUMMARY

When using both three-dimensional medical images such as CT images or MRI images and two-dimensional medical images such as X-ray images, respective coordinate systems must be matched to determine an exact location of an affected area of a subject. However, when a doctor performs a surgery by referencing pre-captured three-dimensional medical images, whether or not a surgical tool is accurately positioned in the affected area relies on the doctor's discretionary judgment.

One embodiment of the present disclosure may include, a system for determining a location of a subject or a surgical tool, the system comprising: an X-ray device comprising a plurality of X-ray sources configured to irradiate the subject with X-rays and an X-ray detector configured to detect X-rays transmitted through the subject; a memory configured to store respective location coordinates of the plurality of X-ray sources and the X-ray detector in a first coordinate system of the system; and a processor configured to obtain a three-dimensional medical image of the subject from an external device, obtain a plurality of first X-ray images of the subject using the X-ray device, match a second coordinate system of the three-dimensional medical image to the first coordinate system based on the respective location coordinates of the plurality of X-ray sources and the X-ray detector in the first coordinate system, the plurality of first X-ray images, and the three-dimensional medical image, determine location coordinates of the subject in the first coordinate system based on the matching result, obtain a plurality of second X-ray images of the surgical tool comprising an electrode using the X-ray device, and determine location coordinates of the surgical tool in the first coordinate system based on the plurality of second X-ray images.

In one embodiment, the processor is configured to: obtain a plurality of projection images by projecting the three-dimensional medical image onto a two-dimensional plane; compare the plurality of projection images with the plurality of first X-ray images; determine a projection image having a highest similarity to the plurality of first X-ray images from among the plurality of projection images; and match the second coordinate system of the three-dimensional medical image to the first coordinate system based on the plurality of first X-ray images and the determined projection image.

In one embodiment, the plurality of X-ray sources are disposed on a same plane.

In one embodiment, the plurality of X-ray sources are disposed on a straight line at equal intervals, and the plurality of X-ray sources irradiate the subject with X-rays at different angles from each other.

In one embodiment, the plurality of X-ray sources are X-ray sources using carbon nanotubes.

In one embodiment, the X-ray device further comprises one power supply unit configured to supply high voltage to the plurality of X-ray sources.

In one embodiment, the X-ray device further comprises a marker attached to a predetermined location, the system further comprises a tracking sensor configured to track a location of the marker, the memory is configured to store a first coordinate conversion relationship between the plurality of X-ray sources and the X-ray detector and a second coordinate conversion relationship between the plurality of X-ray sources and the marker, and the processor is configured to: obtain location coordinates of the marker in the first coordinate system from the tracking sensor, obtain respective location coordinates of the plurality of X-ray sources and the X-ray detector in the first coordinate system based on the location coordinates of the marker, and store the respective location coordinates of the plurality of X-ray sources and the X-ray detector in the first coordinate system in the memory.

In one embodiment, the X-ray device further comprises: a connection member connected to the plurality of X-ray sources and the X-ray detector; a first rotation unit in which the plurality of X-ray sources are disposed and configured to rotate around a first rotation axis; and a second rotation unit connected to the connection member and configured to rotate around a second rotation axis.

In one embodiment, the marker is attached to a designated location of the connection member of the X-ray device, and the designated location is located within a field of view of the tracking sensor.

In one embodiment, the marker is attached to a designated location of the first rotation unit of the X-ray device, and the designated location is located within a field of view of the tracking sensor.

One embodiment of the present disclosure may include a method for determining a location of a subject or a surgical tool of a system comprising an X-ray device that comprises a plurality of X-ray sources configured to irradiate the subject with X-rays and an X-ray detector configured to detect X-rays transmitted through the subject, a memory, and a processor, the method comprising: receiving a three-dimensional medical image of the subject from an external device; obtaining a plurality of first X-ray images of the subject using the X-ray device; matching a second coordinate system of the three-dimensional medical image to a first coordinate system based on respective location coordinates of the plurality of X-ray sources and the X-ray detector in the first coordinate system of the system, which are stored in the memory, the plurality of first X-ray images, and the three-dimensional medical image; determining location coordinates of the subject in the first coordinate system based on the matching result; obtaining a plurality of second X-ray images of a surgical tool comprising an electrode using the X-ray device; and determining location coordinates of the surgical tool in the first coordinate system based on the plurality of second X-ray images.

In one embodiment, the matching comprises: obtaining a plurality of projection images by projecting the three-dimensional medical image onto a two-dimensional plane; comparing the plurality of projection images with the plurality of first X-ray images; determining a projection image having a highest similarity to the plurality of first X-ray images from among the plurality of projection images; and matching the second coordinate system of the three-dimensional medical image to the first coordinate system based on the plurality of first X-ray images and the determined projection image.

In one embodiment, the plurality of X-ray sources are disposed on a same plane.

In one embodiment, the plurality of X-ray sources are disposed on a straight line at equal intervals, and the plurality of X-ray sources irradiate the subject with X-rays at different angles from each other.

In one embodiment, the plurality of X-ray sources are X-ray sources using carbon nanotubes.

In one embodiment, the X-ray device further comprises one power supply unit configured to supply high voltage to the plurality of X-ray sources.

In one embodiment, the X-ray device further comprises a marker attached to a predetermined location, the system further comprises a tracking sensor configured to track a location of the marker, the memory is configured to store a first coordinate conversion relationship between the plurality of X-ray sources and the X-ray detector and a second coordinate conversion relationship between the plurality of X-ray sources and the marker, and the method further comprises: obtaining location coordinates of the marker in the first coordinate system from the tracking sensor; obtaining respective location coordinates of the plurality of X-ray sources and the X-ray detector in the first coordinate system based on the location coordinates of the marker; and storing the respective location coordinates of the plurality of X-ray sources and the X-ray detector in the first coordinate system in the memory.

In one embodiment, the X-ray device further comprises: a connection member connected to the plurality of X-ray sources and the X-ray detector; a first rotation unit in which the plurality of X-ray sources are disposed and configured to rotate around a first rotation axis; and a second rotation unit connected to the connection member and configured to rotate around a second rotation axis.

In one embodiment, the marker is attached to a designated location of the connection member of the X-ray device, and the designated location is located within a field of view of the tracking sensor.

In one embodiment, the marker is attached to a designated location of the first rotation unit of the X-ray device, and the designated location is located within a field of view of the tracking sensor.

The system according to various embodiments of the present disclosure may accurately identify a location of the subject in a three-dimensional spatial coordinate system by matching the three-dimensional medical image of the subject to the coordinate system of the two-dimensional X-ray image of the subject.

The system according to various embodiments of the present disclosure may accurately identify a location of the surgical tool in the three-dimensional spatial coordinate system by capturing an X-ray image of the surgical tool.

According to various embodiments of the present disclosure, since a plurality of X-ray sources are X-ray sources using digital carbon nanotubes, it is possible to shorten the capturing time and the X-ray exposure time of the subject, and reduce the power consumption.

### BRIEF DESCRIPTION OF DRAWINGS

FIG. 1 is a block diagram of a system according to an embodiment of the present disclosure.
FIG. 2A is a diagram illustrating an X-ray device according to an embodiment of the present disclosure.
FIG. 2B is a diagram schematically illustrating a plurality of X-ray sources and an X-ray detector of an X-ray device.
FIGS. 3A and 3B are diagrams illustrating a system according to an embodiment of the present disclosure.
FIG. 4 is a flowchart illustrating an operation of a processor according to an embodiment of the present disclosure.
FIG. 5 is a flowchart illustrating an operation of a processor according to an embodiment of the present disclosure.
FIG. 6 is an X-ray image of a surgical tool according to an embodiment of the present disclosure.
FIG. 7 is a diagram illustrating a location of a surgical tool on a first coordinate system.
FIG. 8 is a flowchart illustrating an operation of a processor according to an embodiment of the present disclosure.
FIGS. 9 to 11 are diagrams illustrating a process of obtaining a first coordinate conversion relationship.
FIGS. 12 to 14 are diagrams illustrating a method for obtaining a second coordinate conversion relationship according to an embodiment of the present disclosure.

### DETAILED DESCRIPTION

Embodiments of the present disclosure are illustrated for describing the technical idea of the present disclosure. The scope of the claims according to the present disclosure is not limited to the embodiments described below or to the detailed descriptions of these embodiments.

All technical or scientific terms used in the present disclosure have meanings that are generally understood by a person having ordinary knowledge in the art to which the present disclosure pertains, unless otherwise specified. The terms used in the present disclosure are all selected only for more clear description of the present disclosure, and are not intended to limit the scope of claims according to the present disclosure.

The expressions "include," "provided with," "have" and the like used herein should be understood as open-ended terms connoting the possibility of inclusion of other embodiments, unless otherwise mentioned in a phrase or sentence including the expressions.

In the present disclosure, the singular of an expression may include the meaning of the plural of the expression unless the context clearly indicates otherwise, and the same applies to singular forms of expressions as set forth in the claims.

The terms "first," "second," etc. used in the present disclosure are used to distinguish multiple components from one another, and are not intended to limit the order or importance of the relevant components.

The term "unit" used in these embodiments means a software component or hardware component, such as a field-programmable gate array (FPGA) and an application specific integrated circuit (ASIC). However, a "unit" is not limited to software and hardware, and it may be configured to be an addressable storage medium or may be configured to run on one or more processors. For example, a "unit" may include components, such as software components, object-oriented software components, class components, and task components, as well as processors, functions, attributes, procedures, subroutines, segments of program codes, drivers, firmware, micro-codes, circuits, data, databases, data structures, tables, arrays, and variables.

The expression "based on" used herein is used to describe one or more factors that influences a decision, an action of judgment or an operation described in a phrase or sentence including the relevant expression, and this expression does not exclude additional factor influencing the decision, the action of judgment or the operation.

In the present disclosure, the expression that a component (e.g., a first component) is "connected" or "coupled" to another component (e.g., a second component) may mean that the first component is connected or coupled to the second component not only directly, but also via another new component (e.g., a third component).

Although process steps, method steps, algorithms, and the like are illustrated in a sequential order in the present disclosure, such processes, methods, and algorithms may be configured to operate in any suitable order. In other words, the steps of the processes, methods, and algorithms described in various embodiments of the present disclosure need not be performed in the order described in various embodiments of the present disclosure. In addition, although some steps are described as being performed non-simultaneously, in other embodiments, such steps may be performed simultaneously. In addition, exemplification of a process in a drawing does not imply that the exemplified process excludes any changes and modifications thereto, that the exemplified process or any steps thereof are essential to one or more of the various embodiments of the present disclosure, or that the exemplified process is desirable.

Hereinafter, embodiments of the present disclosure will be described with reference to the accompanying drawings. In the accompanying drawings, identical or corresponding components are indicated by identical reference numerals. In the following description of embodiments, redundant descriptions of the identical or corresponding components will be omitted. However, the omission of a description of a component does not imply that such a component is not included in an embodiment.

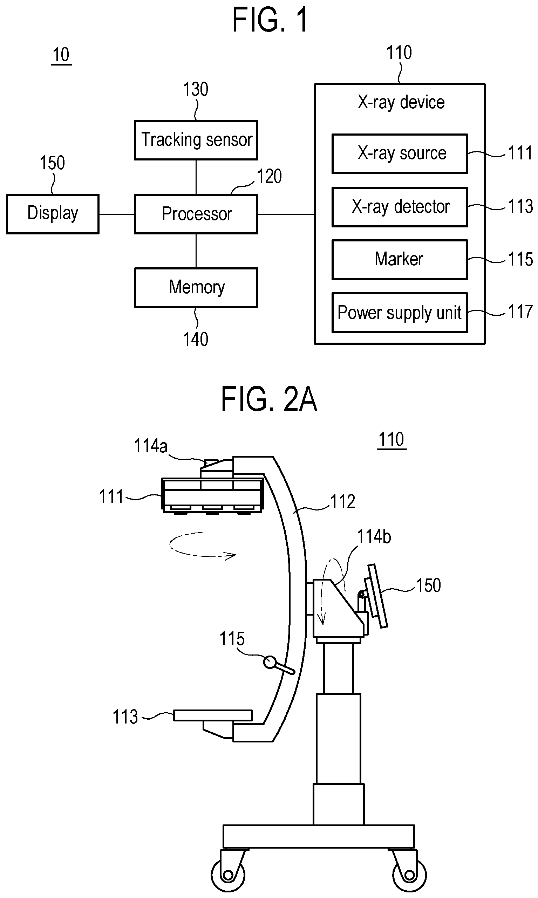

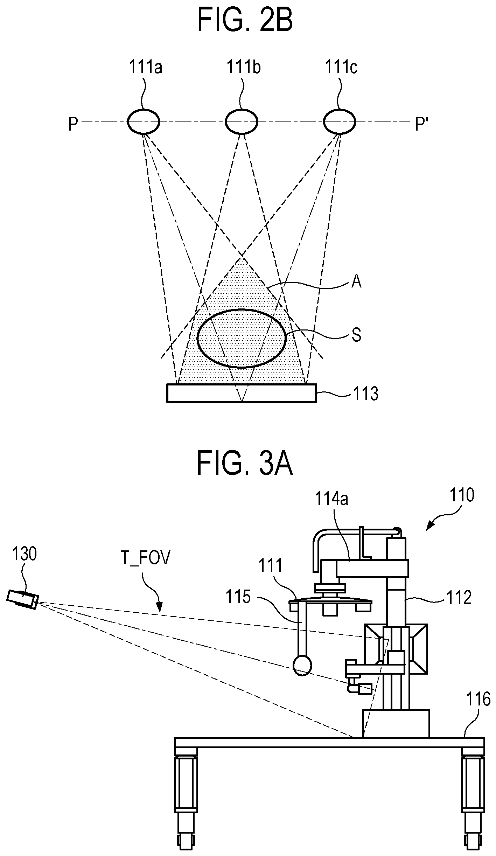

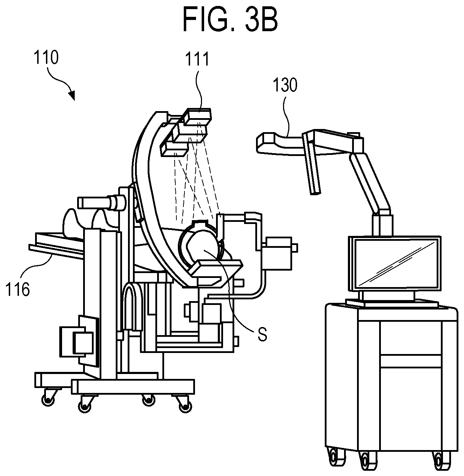

FIG. 1 is a block diagram of a system 10 according to an embodiment of the present disclosure. FIG. 2A is a diagram illustrating an X-ray device 110 according to an embodiment of the present disclosure, and FIG. 2B is a diagram schematically illustrating a plurality of X-ray sources 111 and an X-ray detector 113 of the X-ray device 110. FIGS. 3A and 3B are diagrams illustrating the system 10 according to an embodiment of the present disclosure.

The system 10 for determining a location of a subject or surgical tool may include the X-ray device 110, a processor 120, a tracking sensor 130, and/or a memory 140. In an embodiment, the system 10 may further include a display 150. Even if some of the elements illustrated in FIG. 1 are omitted or replaced, there will be no problem in implementing various embodiments disclosed in this document.

The X-ray device 110 may be a device for irradiating a subject S with an X-ray to obtain an image of the inside of the subject and analyzing the subject. As shown in FIG. 2A, the X-ray device 110 may include X-ray sources 111 and/or an X-ray detector 113. In an embodiment, the X-ray device 110 may further include a marker 115 and/or a power supply unit 117. The marker 115 may be tracked by a tracking sensor 130 to be described later.

The X-ray source 111 of the X-ray device 110 is a light source capable of emitting X-rays and may irradiate the subject with X-rays under the control of the processor 120. The subject may be located between the X-ray source 111 and the X-ray detector 113. In an embodiment, the X-ray device 110 may include a plurality of X-ray sources 111. For example, the X-ray device 110 may include three X-ray sources 111, two X-ray sources 111, or four or more X-ray sources 111. Hereinafter, although a description will be made based on three X-ray sources 111 included for convenience of explanation, the number of X-ray sources 111 is not limited thereto. The plurality of X-ray sources 111 may be X-ray sources 111 using, for example, carbon nanotubes (CNTs). The plurality of X-ray sources 111 may be configured as, for example, a digital X-ray tube with a cold cathode structure using carbon nanotubes.

The X-ray detector 113 of the X-ray device 110 may be a detection device that detects an amount of X-ray or intensity of X-ray. The X-ray detector 113 may detect the amount of X-rays that have passed through the subject among the X-rays radiated onto the subject from the X-ray source 111. If the internal density of the subject is not uniform, the amount of X-rays absorbed by the subject may vary depending on a direction in which the X-rays are radiated. The X-ray detector 113 may measure the amount of X-rays reduced as X-rays radiated at various angles pass through the subject, and the processor 120 may produce a two-dimensional X-ray image projecting the inside of the subject based on the value measured by the X-ray detector 113. For example, the processor 120 may convert the X-rays measured by the X-ray detector 113 into visible light, and then convert it into a digital signal, thereby producing an image. The X-ray detector 113 may be configured in the form of a plate, or may be implemented in various forms capable of detecting X-rays.

The plurality of X-ray sources 111 may be disposed on a single plane. For example, as shown in FIG. 2B, the plurality of X-ray sources 111 may all be disposed on a single plane (plane P-P') parallel to the surface of the X-ray detector 113. The plurality of X-ray sources 111 may be disposed on a straight line at equal intervals, or may be disposed on a straight line at different intervals. For example, the interval between the plurality of X-ray sources 111 may be determined to be 50 to 200 nm. For example, the interval between the plurality of X-ray sources 111 may be 150 nm. The irradiating angle may be configured to differ between the plurality of X-ray sources 111 such that the subject may be located within the field of view of the X-ray beams radiated from all of the plurality of X-ray sources 11. For example, as shown in FIG. 2B, among the plurality of X-ray sources 111, a first X-ray source 111a located on the left may be configured to radiate an X-ray tilted to the right at an angle of 15 degrees with respect to the normal line of the corresponding plane (plane P-P'), a second X-ray source 111b located at the center may be configured to radiate an X-ray parallel to the normal line of the corresponding plane (plane P-P'), and a third X-ray source 111c located on the right may be configured to radiate an X-ray tilted to the left at an angle of 15 degrees with respect to the normal line of the corresponding plane (plane P-P'). In the above case, the subject S may be located in an area A where the fields of view of the plurality of X-ray sources 111 overlap each other. The above-described angle is exemplary, and it is obvious that the plurality of X-ray sources 111 may be configured at various angles capable of irradiating the subject with X-rays. If the plurality of X-ray sources 111 are disposed as described above, for example, even if the plurality of X-ray sources 111 are not disposed on a rotating plane, a plurality of X-ray images of the subject captured from different directions may be obtained.

The respective X-ray sources 111 may be selectively and sequentially driven at a constant time interval (e.g., several ms). For example, the processor 120 may first drive the first X-ray source 111a among the plurality of X-ray sources 111 and stop the same, then drive the second X-ray source 111b after a predetermined time elapses (e.g., after 5 ms) and stop the same, and then drive the third X-ray source 111c after the predetermined time elapses and then stop the same. That is, the processor 120 may control the plurality of X-ray sources 111 to be driven selectively and sequentially within a short period of time, instead of driving them simultaneously, thereby reducing the exposure amount of the subject to the X-rays.

The X-ray device 110 according to various embodiments may further include a connection member 112, a first rotation unit 114a, a second rotation unit 114b, and/or a stage 116. The connection member 112 may be a configuration for connecting the plurality of X-ray sources 111 and the X-ray detector 113. For example, the connection member 112 may be a C-shaped robot arm. The first rotation unit 114a may have a plurality of X-ray sources 111 disposed thereon and may be configured to rotate around a first rotation axis. For example, the first rotation unit 114a may be configured to yaw-rotate around the z-axis. As the first rotation unit 114a yaw-rotates, the plurality of X-ray sources 111 may be yaw-rotated, thereby obtaining a vertical X-ray image or a horizontal X-ray image of the subject.

The second rotation unit 114b of the X-ray device 110 may be connected to the connection member 112 and configured to rotate around a second rotation axis. For example, the second rotation unit 114b may be configured to roll-rotate around the x-axis. That is, as the second rotation unit 114b roll-rotates, the plurality of X-ray sources 111 and the X-ray detector 113 may be rotated together. A user (e.g., a doctor) may adjust the locations of the first rotation unit 114a and the second rotation unit 114b to obtain accurate X-ray images of the subject.

The subject S may be located on the stage 116 of the X-ray device 110. The stage 116 may move in the x-axis direction. After the subject S is located on the stage 116, the stage 116 may move between the plurality of X-ray sources 111 and the X-ray detector 113. For example, as shown in FIG. 3B, the subject S may be located on the stage 116, so that the surgical site of the subject S may be located within the field of view T_FOV of the plurality of X-ray sources 111. The power supply unit 117 of the X-ray device 110 may supply power required to operate respective elements of the X-ray device 110. The power supply unit 117 may supply power required for the plurality of X-ray sources 111 to output X-rays.

The tracking sensor 130 may be a device for tracking the location and/or posture of a subject. For example, the tracking sensor 130 may measure the location and/or posture of a marker 115 attached to a target (e.g., the X-ray device 110), thereby tracking the target to which the marker 115 is attached. The marker 115 may generate energy or signals so that the tracking sensor 130 is able to sense the same. The marker 115 may be attached to a predetermined location of the X-ray device 110. The marker 115 may be attached to various locations of the respective elements of the X-ray device 110. According to an embodiment, the marker 115 may be attached to a designated location of the connection member 112 of the X-ray device 110 as illustrated in FIG. 2A, or may be attached to a designated location of the first rotation unit 114a of the X-ray device 110 as illustrated in FIG. 3A. Here, the designated location falls within the field of view of the tracking sensor 130. For example, as illustrated in FIG. 3A, the marker 115 is located within the field of view T_FOV of the tracking sensor 130.

The tracking method using the tracking sensor 130 is not particularly limited, but, in general, an optical tracking method based on optical technology or an electromagnetic tracking method based on electromagnetic technology may be used. In addition, various tracking methods may be used in combination.

The location measured by the tracking sensor 130 may be defined as three-dimensional spatial coordinates, such as coordinates on the x-, y-, and z-axes of an orthogonal coordinate system. In addition, the posture measured by the tracking sensor 130 may be defined as rotation information, such as roll, pitch, and yaw. In order to accurately track an object, 6 degrees of freedom of the location and posture of the object defined above may be measured. According to an embodiment, the tracking sensor 130 may measure the location of the marker 115 attached to a designated location of the X-ray device 110, thereby identifying the location of the X-ray device 110.

The processor 120 may be a configuration capable of performing computations or data processing related to control and/or communication of respective elements included in the system 10. The processor 120 may be operatively connected to the elements of the X-ray device 110, such as the tracking sensor 130, the memory 140, and/or the display 150. The processor 120 may load instructions or data received from other elements of the X-ray device 110 into the memory 140, process instructions or data stored in the memory 140, and store result data.

The memory 140 may store instructions for the operation of the processor 120. The memory 140 may store a first coordinate conversion relationship between the plurality of X-ray sources 111 and the X-ray detector 113, and a second coordinate conversion relationship between the plurality of X-ray sources 111 and the marker 115. For example, the first coordinate conversion relationship and the second coordinate conversion relationship may be matrix vectors. The memory 140 may store respective location coordinates of the plurality of X-ray sources 111 and the X-ray detector 113 in the first coordinate system of the system 10.

The display 150 may display various screens based on the control of the processor 120. The display 150 may display, for example, a three-dimensional medical image or a two-dimensional X-ray image.

The processor 120 may obtain location coordinates of a marker 115 in the first coordinate system of the system 10 through the tracking sensor 130. Here, the first coordinate system may be a three-dimensional spatial coordinate system used in the system 10. The processor 120 may obtain location coordinates of the plurality of X-ray sources 111 and X-ray detector 113 in the first coordinate system based on the location coordinates of the marker 115. For example, the processor 120 may obtain location coordinates of the plurality of X-ray sources 111 based on the second coordinate conversion relationship between the plurality of X-ray sources 111 and the marker 115 and the location coordinates of the marker 115. Thereafter, the processor 120 may obtain location coordinates of the X-ray detector 113 based on the first coordinate conversion relationship between the plurality of X-ray sources 111 and the X-ray detector 113 and the location coordinates of the plurality of X-ray sources 111. Here, the location coordinates of the plurality of X-ray sources 111 and the location coordinates of the X-ray detector 113 are location coordinates defined in the first coordinate system of the system 10. The processor 120 may store the obtained location coordinates of the plurality of X-ray sources and X-ray detector in the memory 140, respectively.

The processor 120 may obtain a three-dimensional medical image of the subject from an external device. The user may obtain a three-dimensional medical image (e.g., an MRI image or a CT image) of the subject using an MRI device or a CT device before surgery, and the processor 120 may receive the three-dimensional medical image of the subject from the MRI device or the CT device. For example, the processor 120 may receive the three-dimensional medical image of the subject from the MRI device or CT device connected through wired or wireless communication. According to another embodiment, the processor 120 may receive the three-dimensional medical image of the subject from a server device. In the above case, the user may upload the three-dimensional medical image of the subject to the server device, and then transmit the three-dimensional medical image of the subject to the system 10.

The processor 120 may obtain a plurality of first X-ray images of the subject using the X-ray device 110. The processor 120 may sequentially drive the plurality of X-ray sources 111 of the X-ray device 110 to obtain a plurality of first X-ray images of the subject. For example, the processor 120 may obtain a plurality of first X-ray images using the plurality of X-ray sources 111, respectively, and in the above case, the plurality of first X-ray images may be X-ray images obtained by photographing the subject from different directions.

The processor 120 may match the second coordinate system of the three-dimensional medical image to the first coordinate system based on the three-dimensional medical image of the subject and the plurality of first X-ray images. For example, the processor 120 may match the second coordinate system of the three-dimensional medical image to the first coordinate system based on the respective location coordinates of the plurality of X-ray sources 111 and the X-ray detector 113 in the first coordinate system, the plurality of first X-ray images of the subject, and the three-dimensional medical image. The second coordinate system of the three-dimensional medical image may denote a coordinate system used to indicate the locations of respective points of the subject expressed in three dimensions on the three-dimensional medical image. The three-dimensional medical image of the subject may indicate the location of the subject in the second coordinate system. A detailed method of matching the second coordinate system of the three-dimensional medical image to the first coordinate system of the system 10 using the three-dimensional medical image and the X-ray image will be described later. The processor 120 may determine the location of the subject in the first coordinate system based on the matching result. That is, the locational relationship of the plurality of X-ray sources 111 and the X-ray detector 113 may be defined in the first coordinate system through a predetermined calibration process, and the second coordinate system of the three-dimensional medical image of the subject may be matched to the first coordinate system based on a plurality of first X-ray images of the subject obtained from the plurality of X-ray sources 111 and the X-ray detector 113. Accordingly, the location of the subject may be accurately determined in the first coordinate system, which is three-dimensional spatial coordinates, using only the X-ray images of the subject.

The processor 120 may also determine location coordinates of the surgical tool using the matching result. The processor 120, using the X-ray device 110, may obtain a plurality of second X-ray images of the surgical tool including an electrode during surgery. The processor 120 may sequentially drive the plurality of X-ray sources 111 of the X-ray device 110 to obtain a plurality of second X-ray images of the surgical tool. The processor 120 may determine the location of the surgical tool in the first coordinate system based on the plurality of second X-ray images. In the above case, since the X-ray images are matched to the first coordinate system, the location of the electrode of the surgical tool indicated on the X-ray images may be used to determine the location of the electrode of the surgical tool in the first coordinate system.

Although the X-ray device 110, the processor 120, the tracking sensor 130, the memory 140, and the display 150 are illustrated as separate elements in FIG. 1, they are not limited thereto. According to an embodiment, the processor 120, the memory 140, and the display 150 may be implemented as a single device by being integrated with the X-ray device 110. According to an embodiment, the processor 120, the tracking sensor 130, the memory 140, and the display 150 may be implemented as a single electronic device separate from the X-ray device 110. According to an embodiment, the processor 120, the memory 140, and the display 150 may be implemented as a separate electronic device separate from the X-ray device 110, and the tracking sensor 130 may also be implemented as a separate imaging device to be communicatively connected to each other.

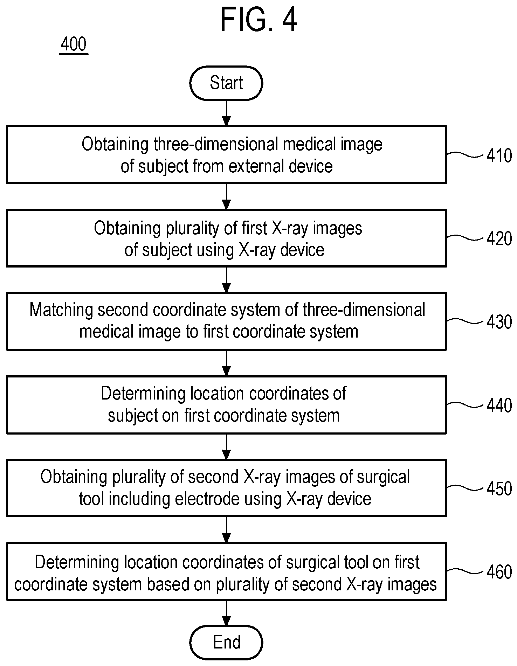

FIG. 4 is a flowchart illustrating the operation of a processor 120 according to various embodiments of the present disclosure. Referring to the flowchart 400, in operation 410, the processor 120 according to various embodiments may receive a three-dimensional medical image of a subject from an external device. The three-dimensional medical image of the subject may be an MRI image or a CT image of the subject obtained in advance before surgery. The external device may be, for example, an MRI device, a CT device, or a server device.

In operation 420, the processor 120 according to various embodiments may obtain a plurality of first X-ray images of the subject using the X-ray device 110. The processor 120 may sequentially and selectively drive the plurality of X-ray sources 111 to obtain a plurality of first X-ray images of the subject. The plurality of first X-ray images may be X-ray images of the subject captured from different directions.

In operation 430, the processor 120 according to various embodiments may match the second coordinate system of the three-dimensional medical image to the first coordinate system. The processor 120 may match the second coordinate system of the three-dimensional medical image to the first coordinate system based on the location coordinates of the plurality of X-ray sources 111 and the X-ray detector 113 in the first coordinate system, the plurality of first X-ray images of the subject, and the three-dimensional medical image. The method of matching the coordinate system using the three-dimensional medical image and the X-ray images may be performed in various ways. For example, the processor 120 may virtually obtain a projection image in which the three-dimensional medical image obtained in advance before the surgery is projected onto a two-dimensional plane. This projection image may be a DRR (digitally reconstructed radiograph) image. The processor 120 may perform coordinate system matching by comparing the produced virtual projection image with the actual X-ray images.

Specifically, the processor 120 may obtain a plurality of projection images in which the three-dimensional medical image is projected in different directions, and compare the plurality of projection images with the plurality of first X-ray images. The processor 120 may determine the projection image with a highest similarity to the plurality of first X-ray images from among the plurality of projection images. In other words, the processor 120 may find the projection image that is most similar to the X-ray images of the actual subject. Afterwards, if the processor 120 finds the projection image that is most similar to the X-ray images, the processor 120 may estimate the location and posture at which the subject of the X-ray images is disposed in the three-dimensional space based on the projection angle or the like when the projection image is produced. In an embodiment, the processor 120 may select a feature point on the X-ray images, calculate an approximate location thereof, and find a projection image that is most similar to the actual X-ray images based on a virtual projection image around the location. In the above case, it is possible to reduce the time required to find a matching projection image. According to an embodiment, although the above-described matching process between the X-ray images and the three-dimensional medical image may be possible using only one X-ray image, the coordinate system matching process performed using two or more X-ray images may result in a more precise matching result.

In operation 440, the processor 120 according to various embodiments may determine the location of the subject in the first coordinate system, based on the matching result. The location and/or posture of the subject may be determined in the first coordinate system through the process of matching the second coordinate system of the three-dimensional medical image to the first coordinate system of the system 10 using the three-dimensional medical image and the plurality of first X-ray images described above. That is, the user may accurately identify the location of the subject, shown in the three-dimensional medical image, in the first coordinate system.

In operation 450, the processor 120 according to various embodiments may obtain a plurality of second X-ray images for a surgical tool including an electrode using the X-ray device 110. For example, the processor 120 may sequentially and selectively drive a plurality of X-ray sources 111 to obtain a plurality of second X-ray images for the surgical tool.

In operation 460, the processor 120 according to various embodiments may determine the location of the surgical tool in the first coordinate system based on the plurality of second X-ray images. Since it is possible to determine the location in the first coordinate system corresponding to a specific location on the X-ray images through the matching operation performed in operation 450 of FIG. 4, the location of the surgical tool in the first coordinate system corresponding to the location of the electrode of the surgical tool shown in the plurality of second X-ray images may be determined. The user (e.g., the doctor) may identify the location of the surgical tool in the first coordinate system by capturing the X-ray images during the surgery, thereby performing an accurate surgery.

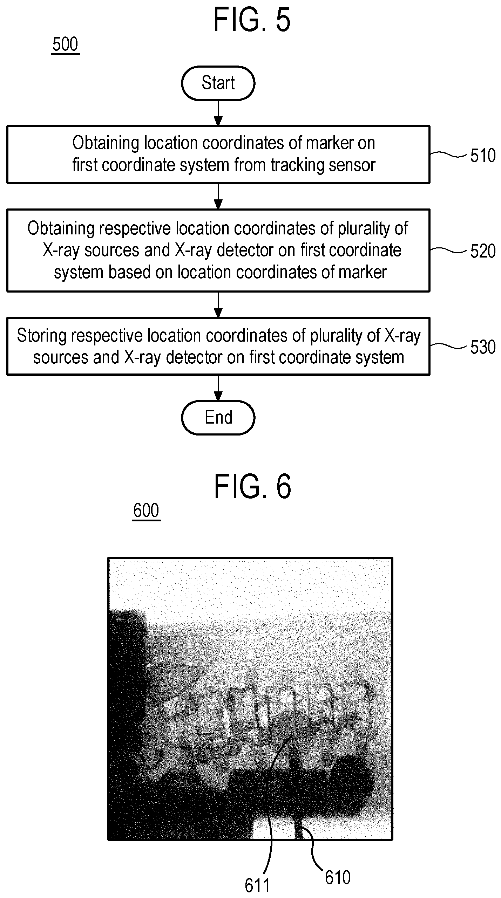

FIG. 5 is a flowchart illustrating an operation of the processor 120 according to various embodiments of the present disclosure. Referring to the flowchart 500, in operation 510, the processor 120 according to various embodiments may obtain location coordinates of the marker 115 in the first coordinate system from the tracking sensor 130. The tracking sensor 130 may obtain the location coordinates of the marker 115 in the first coordinate system by measuring the location of the marker 115 attached to a predetermined location of the X-ray device 110. Here, the first coordinate system may be a three-dimensional spatial coordinate system used in the system 10.

The processor 120 according to various embodiments, in operation 520, may obtain respective location coordinates of the plurality of X-ray sources 111 and the X-ray detector 113 in the first coordinate system based on the obtained location coordinates of the marker 115. Here, the location coordinates of the plurality of X-ray sources 111 may indicate the respective location coordinates of the plurality of X-ray sources 111. The memory 140 stores a first coordinate conversion relationship between the plurality of X-ray sources 111 and the X-ray detector 113 and a second coordinate conversion relationship between the plurality of X-ray sources 111 and the marker 115. The coordinate conversion relationships may be represented as matrix vectors. For example, the processor 120 may obtain the location coordinates of the plurality of X-ray sources 111 based on the location coordinates of the marker 115 and the second coordinate conversion relationship, and obtain the location coordinates of the X-ray detector 113 based on the obtained location coordinates of the plurality of X-ray sources 111 and the first coordinate conversion relationship. A detailed method of obtaining the first coordinate conversion relationship and the second coordinate conversion relationship will be described later.

The processor 120 according to various embodiments may store the location coordinates of the plurality of X-ray sources 111 and the X-ray detector 113 in the first coordinate system in the memory 140 in operation 530. The stored location coordinates of the plurality of X-ray sources 111 and the X-ray detector 113 may be used in the process of matching the second coordinate system of a three-dimensional medical image of the subject to the first coordinate system of the system 10.

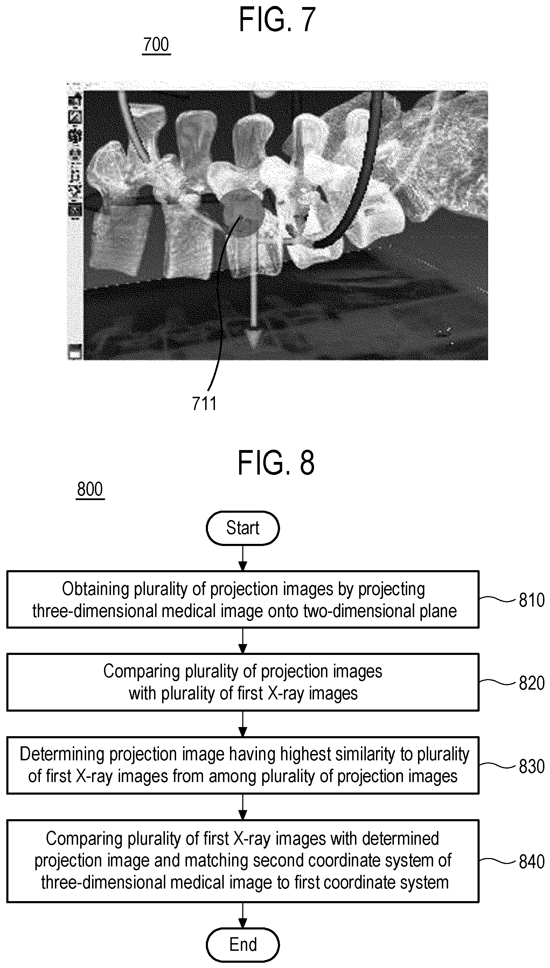

FIG. 6 is an X-ray image 600 of a surgical tool 610 according to various embodiments of the present disclosure, and FIG. 7 is a diagram illustrating a location of a surgical tool in a three-dimensional image 700 having the first coordinate system.

Referring to FIG. 6, the user may capture a two-dimensional X-ray image 600 of the surgical tool 610 using the X-ray device 110 in order to identify whether or not the surgical tool 610 is accurately inserted into the affected area of the subject during surgery. The surgical tool 610 including the electrode 611 is displayed on the X-ray image 600. Thereafter, the processor 120 may determine which location in the three-dimensional first coordinate system corresponds to the location of the surgical tool 610 on the two-dimensional X-ray image 600. Specifically, since the second coordinate system of the three-dimensional medical image is matched to the first coordinate system of the subject using the X-ray image and the three-dimensional medical image of the subject through the matching operation performed in operation 430 of FIG. 4, the processor 120 may determine the location in the first coordinate system corresponding to the location of the surgical tool 610 on the X-ray image 600. FIG. 7 illustrates the location of the surgical tool and its electrode 711 in the three-dimensional image 700. For example, the location of the electrode 711 of the surgical tool in the first coordinate system corresponding to the location of the electrode 611 of the surgical tool 610 in the X-ray image 600 may be determined in the three-dimensional image 700. Therefore, the exact location of the electrode 711 of the surgical tool in the three-dimensional image 700 having the three-dimensional first coordinate system may be identified using only the X-ray image 600 of the surgical tool 610.

FIG. 8 is a flowchart illustrating an operation of a processor 120 according to various embodiments of the present disclosure. Specifically, a flowchart 800 is a detailed flowchart regarding operation 430 in FIG. 4.

In operation 810, the processor 120 according to various embodiments may obtain a plurality of projection images by projecting a three-dimensional medical image onto a two-dimensional plane. The plurality of projection images may be two-dimensional images obtained assuming that the three-dimensional medical image is projected onto a two-dimensional plane. The projection images may be DRR images.

In operation 820, the processor 120 according to various embodiments may compare the plurality of projection images with the plurality of first X-ray images.

In operation 830, the processor 120 according to various embodiments may determine a projection image having the highest similarity to the plurality of first X-ray images from among the plurality of projection images. If the processor 120 finds a projection image with the highest similarity to the X-ray image, the processor 120 may estimate the location and posture at which the subject of the X-ray images is disposed in the three-dimensional space based on the projection angle or the like when the projection image is produced.

In operation 840, the processor 120 according to various embodiments may compare the plurality of first X-ray images with the determined projection image, thereby matching the second coordinate system of the three-dimensional medical image to the first coordinate system. That is, the processor 120 may determine the location of the subject in the first coordinate system by matching the second coordinate system of the three-dimensional medical image to the first coordinate system of the system 10. Furthermore, the location in the first coordinate system corresponding to the specific location indicated on the X-ray image may also be determined by matching the X-ray image with the determined projection image.

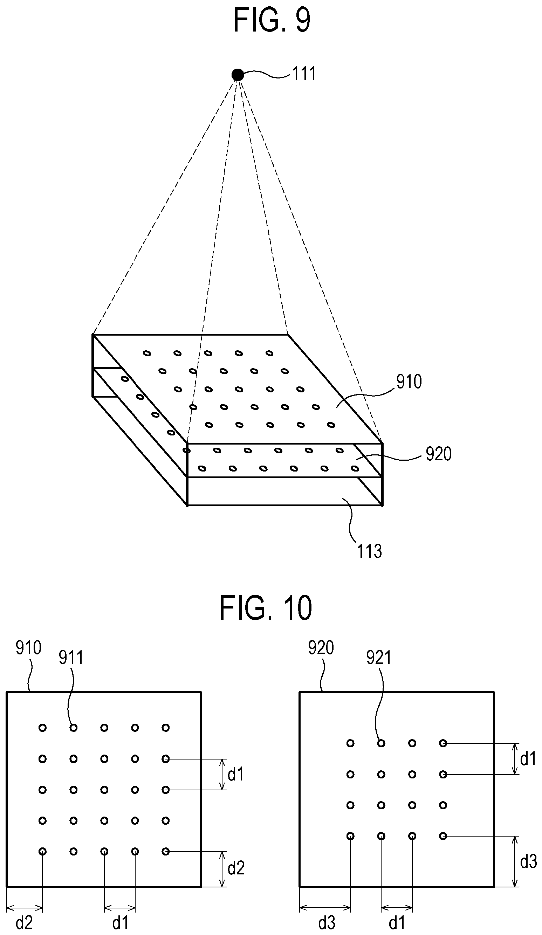

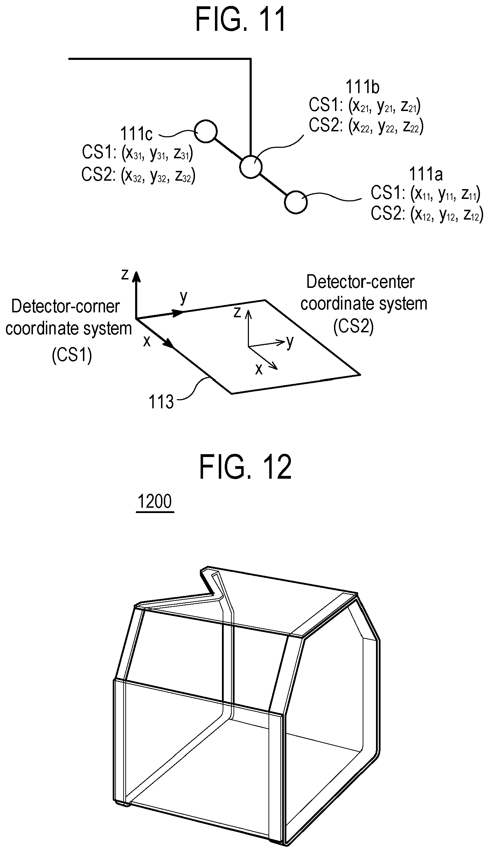

FIGS. 9 to 11 are diagrams illustrating a process of obtaining a first coordinate conversion relationship. Specifically, FIG. 9 is a drawing illustrating a method for obtaining a coordinate relationship between the X-ray sources 111 and the X-ray detector 113, FIG. 10 is a plan view illustrating a calibration tool used for obtaining a coordinate relationship between the X-ray sources 111 and the X-ray detector 113, and FIG. 11 is a drawing illustrating location coordinates of the plurality of X-ray sources 111 based on the X-ray detector 113 coordinate system.

Referring to FIG. 9, a calibration tool may be used to obtain the first coordinate conversion relationship. The calibration tool may include a first calibration plate 910 and a second calibration plate 920. The calibration tool may have a two-layered structure in which the first calibration plate 910 and the second calibration plate 920 are disposed to be spaced a predetermined distance apart from each other, as shown in FIG. 9, which may be placed on the X-ray detector 113. For example, the first calibration plate 910 and the second calibration plate 920 may be disposed in two layers in parallel, and may have a same area. The first calibration plate 910 and the second calibration plate 920 may include a plurality of balls 911 and 921, respectively, arranged in a grid. The plurality of balls 911 and 921 may be metal balls that appear in the X-ray image. The calibration tool may be called, for example, a calibration jig.

The plurality of first balls 911 arranged on the first calibration plate 910 and the plurality of second balls 921 arranged on the second calibration plate 920 are different in number and arrangement from each other. For example, as shown in FIG. 10, the plurality of first balls 911 are arranged in 5*5 on the first calibration plate 910, the distance between the plurality of first balls 911 may be d1, and the distance from the outermost first ball 911 to the edge of the first calibration plate 910 may be d2. The plurality of second balls 921 are arranged in 4*4 on the second calibration plate 920, the distance between the plurality of second balls 921 may be d1, and the distance from the outermost second ball 921 to the edge of the second calibration plate 920 may be d3. In addition, the distance between the first calibration plate 910 and the second calibration plate 920 may be h. The above-described d1, d2, d3, and h are values set by the user.

A plurality of X-ray images for the calibration tool may be obtained using the plurality of X-ray sources 111. For example, when three X-ray sources 111 are used, three X-ray images for the calibration tool may be obtained.

Thereafter, the processor 120 may obtain a coordinate relationship between the X-ray sources 111 and the X-ray detector 113 using a triangulation method. Specifically, the processor 120 may obtain location coordinates of the plurality of X-ray sources 111 based on the X-ray detector 113. Since the coordinate relationship between the X-ray detector 113 and the plurality of X-ray sources 111 is obtained using the existing triangulation method, a detailed description thereof will be omitted.

According to an embodiment, the processor 120 may obtain respective location coordinates of the plurality of X-ray sources 111 on the basis of a detector-center coordinate system based on the X-ray detector 113. According to an embodiment, the processor 120 may obtain respective location coordinates of the plurality of X-ray sources 111 on the basis of a detector-corner coordinate system based on one vertex of the X-ray detector 113. As described above, the coordinate relationship between the X-ray detector 113 and the respective X-ray sources 111 may be obtained using the calibration tool. Therefore, if the processor 120 is aware of the location coordinates of the respective X-ray sources 111, the processor 120 may calculate the location coordinates of the X-ray detector 113 using the above-described coordinate relationship, and vice versa. The above-described coordinate relationship between the X-ray detector 113 and the respective X-ray sources 111 may be represented in a matrix vector or text format.

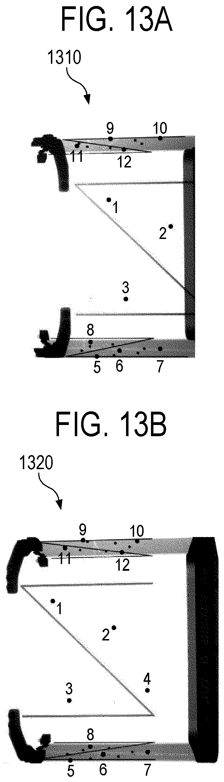

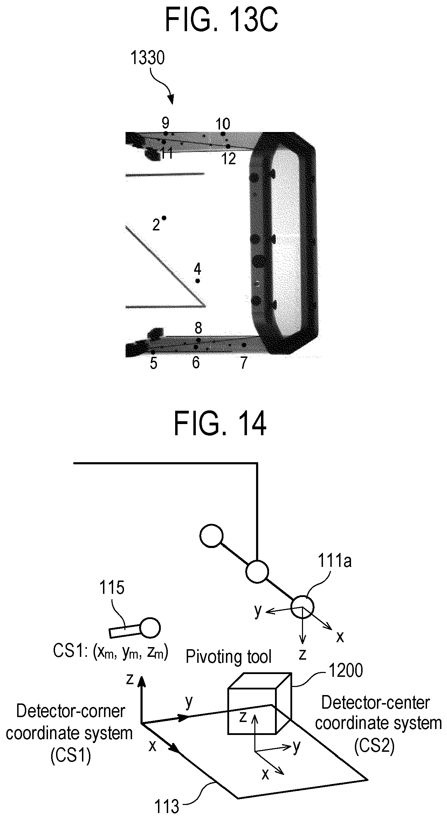

FIGS. 12 to 14 are diagrams illustrating a method for obtaining a second coordinate conversion relationship according to various embodiments of the present disclosure. Specifically, FIG. 12 is a drawing illustrating a pivoting tool 1200 used to obtain the second coordinate conversion relationship, FIGS. 13A, 13B, and 13C are a drawing illustrating a plurality of X-ray images obtained by capturing a calibration phantom using a plurality of X-ray sources 111, and FIG. 14 is a drawing illustrating location coordinates of the marker 115 with respect to the first X-ray source 111.

Referring to FIG. 12, the pivoting tool 1200 may be called a pivoting phantom or a calibration phantom. The pivoting tool 1200 may include a plurality of pivoting balls formed at designated locations. The pivoting balls may be balls made of metal to be detected from the X-ray image, and may also be called pivoting points. For example, a total of twelve pivoting balls may be formed to include four pivoting balls on the upper surface of the pivoting tool, four pivoting balls on the first side, and four pivoting balls on the second side, but the number and locations of the pivoting balls are not limited thereto.

The user may place the pivoting tool 1200 on the X-ray detector 113. Afterwards, the user may obtain a plurality of X-ray images 1310, 1320, and 1330 of the pivoting tool 1200 using the plurality of X-ray sources 111. Since the plurality of X-ray sources 111 respectively radiates X-rays at different angles from different locations, the locations of the pivoting balls indicated in the respective X-ray images 1310, 1320, and 1330 are different from each other.

For example, the processor 120 may extract location coordinates of the plurality of pivoting balls on the X-ray image 1310 obtained by the first X-ray source 111a. The processor 120 may extract location coordinates of the plurality of pivoting balls based on the first X-ray source 111 using, for example, a triangulation method. In the same way, the processor 120 may extract location coordinates of the plurality of pivoting balls on the X-ray images 1320 and 1330 obtained by the second X-ray source 111b and the third X-ray source 111c.

Afterwards, the user may place a marker probe on the plurality of pivoting balls. For example, the marker probe may be located on any one of the plurality of pivoting balls. The marker probe may be a marker that generates energy or signals capable of being sensed by the tracking sensor 130, so it may be sensed by the tracking sensor 130. The marker probe may be a portable and compactly implemented marker.

The processor 120 may obtain location coordinates of the marker probe in the first coordinate system and location coordinates of the marker 115 attached to a designated location of the X-ray device 110 through the tracking sensor 130. Through this, the processor 120 may obtain a locational relationship between the marker 115 attached to a designated location of the X-ray device 110 and the marker probe. Furthermore, since the location coordinates of the marker 115 probe are identical to the location coordinates of the pivoting ball where the marker 115 probe is located, the processor 120 may obtain a locational relationship between the marker 115 attached to a designated location of the X-ray device 110 and the first X-ray source 111. That is, the processor 120 may obtain a coordinate conversion relationship between the first X-ray source 111 and the marker 115. By applying the above-described method to the second X-ray source 111 and the third X-ray source 111, the processor 120 may obtain a first coordinate conversion relationship between the plurality of X-ray sources 111 and the marker 115.

The processor 120 may store the obtained first coordinate conversion relationship and second coordinate conversion relationship in the memory 140. The calibration method described in FIGS. 9 to 14 may be performed once immediately after the system 10 is newly set up, and may be performed whenever the arrangement of the X-ray device 110 is changed. The first coordinate conversion relationship and the second coordinate conversion relationship stored in the memory 140 may be used to match the three-dimensional medical image of the subject to the plurality of first X-ray images of the subject.

Although the method has been described through specific embodiments, the method may also be implemented as computer-readable code on a computer-readable recording medium. The computer-readable recording medium includes any type of storage devices for storing data that can be read by computer systems. Examples of the computer-readable recording medium includes a ROM, a RAM, a CD-ROM, a magnetic tape, a floppy disk, an optical data storage device, etc. Also, the computer-readable recording medium can be distributed to computer systems which are connected through a network so that the computer-readable code can be stored and executed in a distributed manner. Furthermore, functional programs, code and code segments for implementing the foregoing embodiments can be easily inferred by programmers in the art to which the present disclosure pertains.

## Claims

1. A system (10) for determining a location of a subject or a surgical tool, the system (10) comprising:
an X-ray device (110) comprising a plurality of X-ray sources (111, 111a, 111b, 111c) configured to irradiate the subject with X-rays and an X-ray detector (113) configured to detect X-rays transmitted through the subject;
a memory (140) storing respective location coordinates of the plurality of X-ray sources (111, 111a, 111b, 111c) and the X-ray detector (113) in a first coordinate system of the system (10); and
a processor (120) configured to:
obtain a three-dimensional medical image of the subject from an external device;
obtain a plurality of first X-ray images of the subject using the X-ray device (110);
match a second coordinate system of the three-dimensional medical image to the first coordinate system based on the respective location coordinates of the plurality of X-ray sources (111, 111a, 111b, 111c) and the X-ray detector (113) in the first coordinate system, the plurality of first X-ray images, and the three-dimensional medical image;
determine location coordinates of the subject in the first coordinate system based on the matching result;
obtain a plurality of second X-ray images of the surgical tool comprising an electrode (611, 711) using the X-ray device (110); and
determine location coordinates of the surgical tool in the first coordinate system based on the plurality of second X-ray images.

2. The system (10) according to claim 1,
wherein the processor (120) is configured to:
obtain a plurality of projection images by projecting the three-dimensional medical image onto a two-dimensional plane;
compare the plurality of projection images with the plurality of first X-ray images;
determine a projection image having a highest similarity to the plurality of first X-ray images from among the plurality of projection images; and
match the second coordinate system of the three-dimensional medical image to the first coordinate system based on the plurality of first X-ray images and the determined projection image.

3. The system (10) according to claim 1,
wherein the plurality of X-ray sources (111, 111a, 111b, 111c) are disposed on a same plane.

4. The system (10) according to claim 3,
wherein the plurality of X-ray sources (111, 111a, 111b, 111c) are disposed on a straight line at equal intervals, and
wherein the plurality of X-ray sources (111, 111a, 111b, 111c) irradiate the subject with X-rays at different angles from each other.

5. The system (10) according to claim 1,
wherein the plurality of X-ray sources (111, 111a, 111b, 111c) are X-ray sources using carbon nanotubes.

6. The system (10) according to claim 5,
wherein the X-ray device (110) further comprises one power supply unit (117) configured to supply high voltage to the plurality of X-ray sources (111, 111a, 111b, 111c).

7. The system (10) according to claim 1,
wherein the X-ray device (110) further comprises a marker (115) attached to a predetermined location,
wherein the system (10) further comprises a tracking sensor (130) configured to track a location of the marker (115),
wherein the memory (140) is configured to store a first coordinate conversion relationship between the plurality of X-ray sources (111, 111a, 111b, 111c) and the X-ray detector (113) and a second coordinate conversion relationship between the plurality of X-ray sources (111, 111a, 111b, 111c) and the marker (115), and
wherein the processor (120) is configured to:
obtain location coordinates of the marker (115) in the first coordinate system from the tracking sensor (130);
obtain respective location coordinates of the plurality of X-ray sources (111, 111a, 111b, 111c) and the X-ray detector (113) in the first coordinate system based on the location coordinates of the marker (115); and
store the respective location coordinates of the plurality of X-ray sources (111, 111a, 111b, 111c) and the X-ray detector (113) in the first coordinate system in the memory (140).

8. The system (10) according to claim 7, wherein the X-ray device (110) further comprises:
a connection member (112) connected to the plurality of X-ray sources (111, 111a, 111b, 111c) and the X-ray detector (113);
a first rotation unit (114a) in which the plurality of X-ray sources (111, 111a, 111b, 111c) are disposed and configured to rotate around a first rotation axis; and
a second rotation unit (114b) connected to the connection member (112) and configured to rotate around a second rotation axis.

9. The system (10) according to claim 8,
wherein the marker (115) is attached to a designated location of the connection member (112) of the X-ray device (110), and
wherein the designated location is located within a field of view of the tracking sensor (130).

10. The system (10) according to claim 8,
wherein the marker (115) is attached to a designated location of the first rotation unit (114a) of the X-ray device (110), and
wherein the designated location is located within a field of view of the tracking sensor (130).

11. A method for determining a location of a subject or a surgical tool of a system (10) comprising an X-ray device (110) that comprises a plurality of X-ray sources (111, 111a, 111b, 111c) configured to irradiate the subject with X-rays and an X-ray detector (113) configured to detect X-rays transmitted through the subject, a memory (140), and a processor (120), the method comprising:
receiving a three-dimensional medical image of the subject from an external device;
obtaining a plurality of first X-ray images of the subject using the X-ray device (110);
matching a second coordinate system of the three-dimensional medical image to a first coordinate system based on respective location coordinates of the plurality of X-ray sources (111, 111a, 111b, 111c) and the X-ray detector (113) in the first coordinate system of the system (10), which are stored in the memory (140), the plurality of first X-ray images, and the three-dimensional medical image;
determining location coordinates of the subject in the first coordinate system based on the matching result;
obtaining a plurality of second X-ray images of a surgical tool comprising an electrode (611, 711) using the X-ray device (110); and
determining location coordinates of the surgical tool in the first coordinate system based on the plurality of second X-ray images.

12. The method according to claim 11, wherein the matching comprises:
obtaining a plurality of projection images by projecting the three-dimensional medical image onto a two-dimensional plane;
comparing the plurality of projection images with the plurality of first X-ray images;
determining a projection image having a highest similarity to the plurality of first X-ray images from among the plurality of projection images; and
matching the second coordinate system of the three-dimensional medical image to the first coordinate system based on the plurality of first X-ray images and the determined projection image.

13. The method according to claim 11,
wherein the plurality of X-ray sources (111, 111a, 111b, 111c) are disposed on a same plane.

14. The method according to claim 13,
wherein the plurality of X-ray sources (111, 111a, 111b, 111c) are disposed on a straight line at equal intervals, and
wherein the plurality of X-ray sources (111, 111a, 111b, 111c) irradiate the subject with X-rays at different angles from each other.

15. The method according to claim 11,
wherein the plurality of X-ray sources (111, 111a, 111b, 111c) are X-ray sources using carbon nanotubes.

## Patentansprüche

1. System (10) zur Bestimmung einer Position eines Objekts oder eines chirurgischen Instruments, wobei das System (10) umfasst:
eine Röntgeneinrichtung (110), umfassend eine Vielzahl von Röntgenstrahlungsquellen (111, 111a, 111b, 111c), die dazu eingerichtet sind, das Objekt mit Röntgenstrahlen zu bestrahlen, und einen Röntgenstrahlungsdetektor (113), der dazu eingerichtet ist, Röntgenstrahlen zu detektieren, die durch das Objekt transmittiert werden;
einen Speicher (140), der jeweilige Positionskoordinaten der Vielzahl von Röntgenstrahlungsquellen (111, 111a, 111b, 111c) und des Röntgenstrahlungsdetektors (113) in einem ersten Koordinatensystem des Systems (10) speichert; und
einen Prozessor (120), der dazu konfiguriert ist,
ein dreidimensionales medizinisches Bild des Objekts von einer externen Vorrichtung zu erhalten;
eine Vielzahl von ersten Röntgenbildern des Objekts unter Verwendung der Röntgeneinrichtung (110) zu erhalten;
ein zweites Koordinatensystem des dreidimensionalen medizinischen Bilds mit dem ersten Koordinatensystem anhand der jeweiligen Positionskoordinaten der Vielzahl von Röntgenstrahlungsquellen (111, 111a, 111b, 111c) und des Röntgenstrahlungsdetektors (113) in dem ersten Koordinatensystem, der Vielzahl von ersten Röntgenbildern und des dreidimensionalen medizinischen Bilds in Übereinstimmung zu bringen;
Positionskoordinaten des Objekts im ersten Koordinatensystem anhand des Registrierungsergebnisses zu bestimmen;
eine Vielzahl von zweiten Röntgenbildern des chirurgischen Instruments, das eine Elektrode (611, 711) umfasst, unter Verwendung der Röntgeneinrichtung (110) zu erhalten; und
Positionskoordinaten des chirurgischen Instruments im ersten Koordinatensystem anhand der Vielzahl von zweiten Röntgenbildern zu bestimmen.

2. System (10) nach Anspruch 1, wobei der Prozessor (120) dazu konfiguriert ist,
eine Vielzahl von Projektionsbildern durch Projizieren des dreidimensionalen medizinischen Bilds auf eine zweidimensionale Ebene zu erhalten;
die Vielzahl von Projektionsbildern mit der Vielzahl von ersten Röntgenbildern zu vergleichen;
unter der Vielzahl von Projektionsbildern ein Projektionsbild zu bestimmen, das die größte Ähnlichkeit mit der Vielzahl von ersten Röntgenbildern aufweist; und
das zweite Koordinatensystem des dreidimensionalen medizinischen Bilds mit dem ersten Koordinatensystem anhand der Vielzahl von ersten Röntgenbildern und des bestimmten Projektionsbilds in Übereinstimmung zu bringen.

3. System (10) nach Anspruch 1,
wobei die Vielzahl von Röntgenstrahlungsquellen (111, 111a, 111b, 111c) auf einer selben Ebene angeordnet sind.

4. System (10) nach Anspruch 3,
wobei die Vielzahl von Röntgenstrahlungsquellen (111, 111a, 111b, 111c) in gleichen Abständen auf einer geraden Linie angeordnet sind, und
wobei die Vielzahl von Röntgenstrahlungsquellen (111, 111a, 111b, 111c) das Objekt aus voneinander verschiedenen Winkeln mit Röntgenstrahlen bestrahlen.

5. System (10) nach Anspruch 1,
wobei die Vielzahl von Röntgenstrahlungsquellen (111, 111a, 111b, 111c) Röntgenstrahlungsquellen sind, die Kohlenstoffnanoröhren verwenden.

6. System (10) nach Anspruch 5,
wobei die Röntgeneinrichtung (110) ferner eine Stromversorgungseinheit (117) umfasst, die dazu eingerichtet ist, die Vielzahl von Röntgenstrahlungsquellen (111, 111a, 111b, 111c) mit Hochspannung zu versorgen.

7. System (10) nach Anspruch 1,
wobei die Röntgeneinrichtung (110) ferner eine Markierung (115) umfasst, die an einer vorbestimmten Stelle angebracht ist,
wobei das System (10) ferner einen Verfolgungssensor (130) umfasst, der zum Verfolgen einer Position der Markierung (115) eingerichtet ist,
wobei der Speicher (140) dazu konfiguriert ist, eine erste Koordinatenumwandlungsbeziehung zwischen der Vielzahl von Röntgenstrahlungsquellen (111, 111a, 111b, 111c) und dem Röntgenstrahlungsdetektor (113) und eine zweite Koordinatenumwandlungsbeziehung zwischen der Vielzahl von Röntgenstrahlungsquellen (111, 111a, 111b, 111c) und der Markierung (115) zu speichern, und
wobei der Prozessor (120) dazu konfiguriert ist,
Positionskoordinaten der Markierung (115) im ersten Koordinatensystem vom Verfolgungssensor (130) zu erhalten;
jeweilige Positionskoordinaten der Vielzahl von Röntgenstrahlungsquellen (111, 111a, 111b, 111c) und des Röntgenstrahlungsdetektors (113) im ersten Koordinatensystem auf der Grundlage der Positionskoordinaten der Markierung (115) zu erhalten; und
die jeweiligen Positionskoordinaten der Vielzahl von Röntgenstrahlungsquellen (111, 111a, 111b, 111c) und des Röntgenstrahlungsdetektors (113) im ersten Koordinatensystem im Speicher (140) zu speichern.

8. System (10) nach Anspruch 7, wobei die Röntgeneinrichtung (110) ferner umfasst: ein Verbindungselement (112), das mit der Vielzahl von Röntgenstrahlungsquellen (111, 111a, 111b, 111c) und dem Röntgenstrahlungsdetektor (113) verbunden ist;
eine erste Rotationseinheit (114a), in der die Vielzahl von Röntgenstrahlungsquellen (111, 111a, 111b, 111c) angeordnet und dazu eingerichtet sind, sich um eine erste Rotationsachse zu drehen; und
eine zweite Rotationseinheit (114b), die mit dem Verbindungselement (112) verbunden und zum Rotieren um eine zweite Rotationsachse eingerichtet ist.

9. System (10) nach Anspruch 8,
wobei die Markierung (115) an einer bestimmten Stelle des Verbindungselements (112) der Röntgeneinrichtung (110) angebracht ist, und
wobei sich die bestimmte Stelle in einem Sichtfeld des Verfolgungssensors (130) befindet.

10. System (10) nach Anspruch 8,
wobei die Markierung (115) an einer bestimmten Stelle der ersten Rotationseinheit (114a) der Röntgeneinrichtung (110) angebracht ist, und
wobei sich die bestimmte Stelle in einem Sichtfeld des Verfolgungssensors (130) befindet.

11. Verfahren zum Bestimmen einer Position eines Objekts oder eines chirurgischen Instruments eines Systems (10), das eine Röntgeneinrichtung (110), die eine Vielzahl von Röntgenstrahlungsquellen (111, 111a, 111b, 111c), die dazu eingerichtet sind, das Objekt mit Röntgenstrahlen zu bestrahlen, und einen Röntgenstrahlungsdetektor (113), der dazu eingerichtet ist, durch das Objekt transmittierte Röntgenstrahlen zu detektieren, umfasst, einen Speicher (140) und einen Prozessor (120) umfasst, wobei das Verfahren umfasst:
Empfangen eines dreidimensionalen medizinischen Bilds des Objekts von einer externen Vorrichtung;
Erhalten einer Vielzahl von ersten Röntgenbildern des Objekts unter Verwendung der Röntgeneinrichtung (110);
Registrieren eines zweiten Koordinatensystems des dreidimensionalen medizinischen Bilds bezüglich eines ersten Koordinatensystems anhand jeweiliger in dem Speicher (140) gespeicherter Positionskoordinaten der Vielzahl von Röntgenstrahlungsquellen (111, 111a, 111b, 111c) und des Röntgenstrahlungsdetektors (113) in dem ersten Koordinatensystem des Systems (10), der Vielzahl von ersten Röntgenbildern und des dreidimensionalen medizinischen Bilds;
Bestimmen von Positionskoordinaten des Objekts im ersten Koordinatensystem anhand des Registrierungsergebnisses;
Erhalten einer Vielzahl von zweiten Röntgenbildern des chirurgischen Instruments, das eine Elektrode (611, 711) umfasst, unter Verwendung der Röntgeneinrichtung (110); und
Bestimmen von Positionskoordinaten des chirurgischen Instruments im ersten Koordinatensystem anhand der Vielzahl von zweiten Röntgenbildern.

12. Verfahren nach Anspruch 11, wobei das Registrieren umfasst:
Erhalten einer Vielzahl von Projektionsbildern durch Projizieren des dreidimensionalen medizinischen Bilds auf eine zweidimensionale Ebene;
Vergleichen der Vielzahl von Projektionsbildern mit der Vielzahl von ersten Röntgenbildern;
Bestimmen unter der Vielzahl von Projektionsbildern eines Projektionsbilds, das die größte Ähnlichkeit mit der Vielzahl von ersten Röntgenbildern aufweist; und
Registrieren des zweiten Koordinatensystems des dreidimensionalen medizinischen Bilds bezüglich des ersten Koordinatensystems anhand der Vielzahl von ersten Röntgenbildern und des bestimmten Projektionsbilds.

13. Verfahren nach Anspruch 11,
wobei die Vielzahl von Röntgenstrahlungsquellen (111, 111a, 111b, 111c) auf einer selben Ebene angeordnet sind.

14. Verfahren nach Anspruch 13,
wobei die Vielzahl von Röntgenstrahlungsquellen (111, 111a, 111b, 111c) in gleichen Abständen auf einer geraden Linie angeordnet sind, und
wobei die Vielzahl von Röntgenstrahlungsquellen (111, 111a, 111b, 111c) das Objekt aus voneinander verschiedenen Winkeln mit Röntgenstrahlen bestrahlen.

15. Verfahren nach Anspruch 11,
wobei die Vielzahl von Röntgenstrahlungsquellen (111, 111a, 111b, 111c) Röntgenstrahlungsquellen sind, die Kohlenstoffnanoröhren verwenden.

## Revendications

1. Système (10) de détermination d'un emplacement d'un sujet ou d'un outil chirurgical, le système (10) comprenant :
un dispositif de radiographie (110) comprenant une pluralité de sources de rayons X (111, 111a, 111b, 111c) conçues pour irradier le sujet par des rayons X et un détecteur de rayons X (113) conçu pour détecter des rayons X transmis à travers le sujet ;
une mémoire (140) stockant des coordonnées d'emplacement respectives de la pluralité de sources de rayons X (111, 111a, 111b, 111c) et du détecteur de rayons X (113) dans un premier système de coordonnées du système (10) ; et
un processeur (120) configuré pour :
obtenir une image médicale tridimensionnelle du sujet à partir d'un dispositif externe ;
obtenir une pluralité de premières images radiographiques du sujet à l'aide du dispositif de radiographie (110) ;
mettre en correspondance un second système de coordonnées de l'image médicale tridimensionnelle avec le premier système de coordonnées sur la base des coordonnées d'emplacement respectives de la pluralité de sources de rayons X (111, 111a, 111b, 111c) et du détecteur de rayons X (113) dans le premier système de coordonnées, de la pluralité de premières images radiographiques et de l'image médicale tridimensionnelle ;
déterminer des coordonnées d'emplacement du sujet dans le premier système de coordonnées sur la base du résultat de mise en correspondance ;
obtenir une pluralité de secondes images radiographiques de l'outil chirurgical comprenant une électrode (611, 711) à l'aide du dispositif de radiographie (110) ; et
déterminer des coordonnées d'emplacement de l'outil chirurgical dans le premier système de coordonnées sur la base de la pluralité de secondes images radiographiques.

2. Système (10) selon la revendication 1, le processeur (120) étant configuré pour :
obtenir une pluralité d'images de projection par projection de l'image médicale tridimensionnelle sur un plan bidimensionnel ;
comparer la pluralité d'images de projection à la pluralité de premières images radiographiques ;
déterminer une image de projection présentant une similarité la plus élevée avec la pluralité de premières images radiographiques parmi la pluralité d'images de projection ; et
mettre en correspondance le second système de coordonnées de l'image médicale tridimensionnelle avec le premier système de coordonnées sur la base de la pluralité de premières images radiographiques et de l'image de projection déterminée.

3. Système (10) selon la revendication 1,
la pluralité de sources de rayons X (111, 111a, 111b, 111c) étant disposées sur un même plan.

4. Système (10) selon la revendication 3,
la pluralité de sources de rayons X (111, 111a, 111b, 111c) étant disposées sur une ligne droite à intervalles égaux et
la pluralité de sources de rayons X (111, 111a, 111b, 111c) irradiant le sujet par des rayons X selon des angles différents les uns des autres.

5. Système (10) selon la revendication 1,
la pluralité de sources de rayons X (111, 111a, 111b, 111c) étant des sources de rayons X utilisant des nanotubes de carbone.

6. Système (10) selon la revendication 5,
le dispositif de radiographie (110) comprenant en outre une unité d'alimentation électrique (117) configurée pour fournir une haute tension à la pluralité de sources de rayons X (111, 111a, 111b, 111c).

7. Système (10) selon la revendication 1,
le dispositif de radiographie (110) comprenant en outre un marqueur (115) fixé à un emplacement prédéterminé,
le système (10) comprenant en outre un capteur de suivi (130) conçu pour suivre un emplacement du marqueur (115),
la mémoire (140) étant configurée pour stocker une première relation de conversion de coordonnées entre la pluralité de sources de rayons X (111, 111a, 111b, 111c) et le détecteur de rayons X (113) et une seconde relation de conversion de coordonnées entre la pluralité de sources de rayons X (111, 111a, 111b, 111c) et le marqueur (115) et
le processeur (120) étant configuré pour :
obtenir des coordonnées d'emplacement du marqueur (115) dans le premier système de coordonnées à partir du capteur de suivi (130) ;
obtenir des coordonnées d'emplacement respectives de la pluralité de sources de rayons X (111, 111a, 111b, 111c) et du détecteur de rayons X (113) dans le premier système de coordonnées sur la base des coordonnées d'emplacement du marqueur (115) ; et
stocker les coordonnées d'emplacement respectives de la pluralité de sources de rayons X (111, 111a, 111b, 111c) et du détecteur de rayons X (113) dans le premier système de coordonnées dans la mémoire (140).

8. Système (10) selon la revendication 7, le dispositif de radiographie (110) comprenant en outre : un élément de liaison (112) relié à la pluralité de sources de rayons X (111, 111a, 111b, 111c) et au détecteur de rayons X (113) ;
une première unité de rotation (114a) dans laquelle la pluralité de sources de rayons X (111, 111a, 111b, 111c) sont disposées et conçues pour tourner autour d'un premier axe de rotation ; et
une seconde unité de rotation (114b) reliée à l'élément de liaison (112) et conçue pour tourner autour d'un second axe de rotation.

9. Système (10) selon la revendication 8,
le marqueur (115) étant fixé à un emplacement désigné de l'élément de liaison (112) du dispositif de radiographie (110) et
l'emplacement désigné étant situé dans un champ de vision du capteur de suivi (130).

10. Système (10) selon la revendication 8,
le marqueur (115) étant fixé à un emplacement désigné de la première unité de rotation (114a) du dispositif de radiographie (110) et
l'emplacement désigné étant situé dans un champ de vision du capteur de suivi (130).

11. Procédé de détermination d'un emplacement d'un sujet ou d'un outil chirurgical d'un système (10) comprenant un dispositif de radiographie (110) qui comprend une pluralité de sources de rayons X (111, 111a, 111b, 111c) conçues pour irradier le sujet par des rayons X et un détecteur de rayons X (113) conçu pour détecter des rayons X transmis à travers le sujet, une mémoire (140) et un processeur (120), le procédé comprenant :
la réception d'une image médicale tridimensionnelle du sujet à partir d'un dispositif externe ;
l'obtention d'une pluralité de premières images radiographiques du sujet à l'aide du dispositif de radiographie (110) ;
la mise en correspondance d'un second système de coordonnées de l'image médicale tridimensionnelle avec un premier système de coordonnées sur la base de coordonnées d'emplacement respectives de la pluralité de sources de rayons X (111, 111a, 111b, 111c) et du détecteur de rayons X (113) dans le premier système de coordonnées du système (10), qui sont stockées dans la mémoire (140), de la pluralité de premières images radiographiques et de l'image médicale tridimensionnelle ;
la détermination de coordonnées d'emplacement du sujet dans le premier système de coordonnées sur la base du résultat de mise en correspondance ;
l'obtention d'une pluralité de secondes images radiographiques d'un outil chirurgical comprenant une électrode (611, 711) à l'aide du dispositif de radiographie (110) ; et
la détermination de coordonnées d'emplacement de l'outil chirurgical dans le premier système de coordonnées sur la base de la pluralité de secondes images radiographiques.

12. Procédé selon la revendication 11, la mise en correspondance comprenant :
l'obtention d'une pluralité d'images de projection par projection de l'image médicale tridimensionnelle sur un plan bidimensionnel ;
la comparaison de la pluralité d'images de projection à la pluralité de premières images radiographiques ; la détermination d'une image de projection présentant une similarité la plus élevée avec la pluralité de premières images radiographiques parmi la pluralité d'images de projection ; et
la mise en correspondance du second système de coordonnées de l'image médicale tridimensionnelle avec le premier système de coordonnées sur la base de la pluralité de premières images radiographiques et de l'image de projection déterminée.

13. Procédé selon la revendication 11,
la pluralité de sources de rayons X (111, 111a, 111b, 111c) étant disposées sur un même plan.

14. Procédé selon la revendication 13,
la pluralité de sources de rayons X (111, 111a, 111b, 111c) étant disposées sur une ligne droite à intervalles égaux et
la pluralité de sources de rayons X (111, 111a, 111b, 111c) irradiant le sujet par des rayons X selon des angles différents les uns des autres.

15. Procédé selon la revendication 11,
la pluralité de sources de rayons X (111, 111a, 111b, 111c) étant des sources de rayons X utilisant des nanotubes de carbone.
